# EUROPEAN PATENT APPLICATION

(11) **EP 4 289 937 A1**
(43) Date of publication of application: **13.12.2023**
(21) Application number: 22749855.7
(22) Date of filing: 08.02.2022
(51) Int. Cl.: C12N 5/071, A61L 27/38, A61P 1/18, C12N 5/077

(54) **SHEET-SHAPED CELL CULTURE FOR COVERING CUT SURFACE OF PANCREAS**

(30) Priority: 08.02.2021 JP 2021018430
(71) Applicant: NAGASAKI UNIVERSITY, Nagasaki 852-8521 (JP); TERUMO Kabushiki Kaisha, Tokyo 151-0072 (JP)
(72) Inventor: MARUYA, Yasuhiro, Nagasaki-shi, Nagasaki 852-8521 (JP); YAMAGUCHI, Shun, Nagasaki-shi, Nagasaki 852-8521 (JP); KANETAKA, Kengo, Nagasaki-shi, Nagasaki 852-8521 (JP); EGUCHI, Susumu, Nagasaki-shi, Nagasaki 852-8521 (JP); HIGASHI, Miki, Nagasaki-shi, Nagasaki 852-8521 (JP); OHASHI, Fumiya, Ashigarakami-gun, Kanagawa 259-0151 (JP); KAI, Miho, Ashigarakami-gun, Kanagawa 259-0151 (JP); ARAMAKI, Naoki, Ashigarakami-gun, Kanagawa 259-0151 (JP)
(74) Representative: Prüfer & Partner mbB Patentanwälte · Rechtsanwälte
(86) International application number: PCT/JP2022/004813
(87) International publication number: WO 2022/168984

(57) **Abstract**

An object of the invention is to provide a solution for preventing or treating pancreatic fistula.

The above object is achieved by providing a sheet-shaped cell culture for covering a cut surface of a pancreas and a method for preventing or treating pancreatic fistula. In the sheet-shaped cell culture, the cut surface of the pancreas includes cut surfaces of a pancreatic parenchyma and a pancreatic duct, and is connected to an intestinal wall of a small intestine in a liquid-tight manner via the sheet-shaped cell culture. The method for preventing or treating pancreatic fistula includes a step of covering, using a sheet-shaped cell culture, a cut surface of a pancreas that includes cut surfaces of a pancreatic parenchyma and a pancreatic duct, and a step of connecting the cut surface of the pancreas to an intestinal wall of a small intestine in a liquid-tight manner via the sheet-shaped cell culture.

## Description

### Technical Field

The present invention relates to a sheet-shaped cell culture for covering a cut surface of a pancreas.

### Background Art

In recent years, attempts have been made to transplant various cells to repair damaged tissues or the like. For example, use of fetal cardiomyocytes, skeletal myoblast cells, mesenchymal stem cells, cardiac stem cells, ES cells, iPS cells, and the like has been attempted to repair damaged myocardial tissues caused by ischemic heart diseases such as angina pectoris and myocardial infarction (NPL 1).

As a part of the attempts, cell structures formed using scaffolds, and sheet-shaped cell cultures formed by forming cells into a sheet shape have been developed (PTL 1 and NPL 2).

For therapeutic applications of sheet-shaped cell cultures, there are ongoing studies regarding use of a cultured epidermal sheet for skin damage due to a burn injury or the like, use of a corneal epithelial sheet-shaped cell culture for corneal damage, use of an oral mucosal sheet-shaped cell culture for endoscopic resection of esophageal cancer, and the like, and a part thereof is in the stage of clinical applications.

As one of such applications, it has been proposed to use a sheet-shaped cell culture for healing damage of an organ such as a digestive tract. For example, PTL 2 discloses use of a sheet-shaped cell culture containing mesenchymal stem cells to heal or prevent leakage from a damaged portion of a digestive tract caused by suturing failure or the like. Further, NPLs 3 and 4 describe that a skeletal muscle myoblast cell sheet can be used for healing of pancreatic fistula or gastric perforation in an animal model.

The term "pancreatic fistula" refers to the leakage of pancreatic juice from a pancreas, and may be caused by, for example, pancreatitis or a trauma, or may be caused by the occurrence of a through hole at a suture site after a surgical treatment of the pancreas. The leaked pancreatic juice may be activated by being mixed with digestive juice and bile, and may cause an inflammation due to lysing of tissues in a vicinity of a leaking site. In addition, pancreatic fistula may cause a serious prognosis by lysing blood vessels in the vicinity of the leaking site and causing hemorrhage.

PTL 3 describes that an adipose tissue-derived mesenchymal stem cell sheet treated with mannose exhibits an excellent therapeutic effect for pancreatic fistula in a rat model. Specifically, PTL 3 describes that when the mesenchymal stem cell sheet is attached to a pancreas stump of pancreas of the rat in which an incision is made, an amylase value of ascites, which is an indicator of pancreatic fistula, decreases, and a lipase value of the ascites exhibits the same tendency.

On the other hand, in recent years, an attempt has also been made to sandwich a sheet-shaped main body portion that promotes fusing of biological tissues between a vicinity of a cut surface of a pancreatic parenchyma and an intestinal wall of a jejunum, and to join the vicinity of the cut surface of the pancreatic parenchyma and the intestinal wall of the jejunum (PTL 4). In addition, an attempt has also been made to promote fusion between a jejunum, a pancreatic parenchyma and a pancreatic duct by disposing a biodegradable sheet between the jejunum and the pancreatic parenchyma and suturing the jejunum and the pancreatic parenchyma (PTL 5).

### Citation List

### Patent Literature

PTL 1: JP2007-528755A
PTL 2: WO2017/130802
PTL 3: JP6332800B
PTL 4: JP2019-162405A
PTL 5: WO2019/156230

### Non Patent Literature

NPL 1: Haraguchi Y. et al., Stem Cells Transl. Med., 1(2), 136-141 (2012)
NPL 2: Sawa Y. et al., Surg. Today, 42(2), 181-184 (2012)
NPL 3: Tanaka T. et al., J. Gastroenterol., 48(9), 1081-1089 (2013)
NPL 4: Tanaka S. et al., Surg. Today., 47(1), 114-121 (2017)

### Summary of Invention

### Technical Problem

An object of the invention is to provide a solution for preventing or treating pancreatic fistula.

### Solution to Problem

While studying a method for preventing or treating pancreatic fistula, the inventor of the invention has considered that since a liquid-tightness of a suture site is deteriorated due to the occurrence of a through hole in the suture site after a surgical treatment of a pancreas, pancreatic juice can leak from the suture site. Then, as a result of conducted extensive studies to maintain the liquid-tightness of the suture site, the inventor has found that by covering a cut surface of a pancreas that includes cut surfaces of a pancreatic parenchyma and a pancreatic duct using a sheet-shaped cell culture, and connecting the cut surface of the pancreas to an intestinal wall of a small intestine in a liquid-tight manner via the sheet-shaped cell culture, the liquid-tightness of the suture site can be maintained after the surgical treatment of the pancreas, and as a result of further continuing studies based on this finding, the invention has been completed.

That is, the invention relates to the following.
[1] A sheet-shaped cell culture for covering a cut surface of a pancreas, in which the cut surface of the pancreas includes cut surfaces of a pancreatic parenchyma and a pancreatic duct, and is connected to an intestinal wall of a small intestine in a liquid-tight manner via the sheet-shaped cell culture.
[2] The sheet-shaped cell culture according to [1], in which the pancreatic duct is connected to a through hole in the intestinal wall of the small intestine in a liquid-tight manner via the sheet-shaped cell culture.
[3] The sheet-shaped cell culture according to [2], in which by forming a through hole in the sheet-shaped cell culture at a lumen portion of the cut surface of the pancreatic duct, lumina of the pancreatic duct and the small intestine communicate with each other via the through holes in the sheet-shaped cell culture and the intestinal wall of the small intestine.
[4] The sheet-shaped cell culture according to any one of [1] to [3], in which the pancreatic duct is a main pancreatic duct and/or an accessory pancreatic duct.
[5] The sheet-shaped cell culture according to any one of [1] to [4], in which the small intestine is a duodenum or a jejunum.
[6] The sheet-shaped cell culture according to any one of [1] to [5], in which the cut surface of the pancreas, the through hole in the intestinal wall of the small intestine, and/or the through hole in the sheet-shaped cell culture is formed by a surgical treatment.
[7] The sheet-shaped cell culture according to [6], in which the surgical treatment is pancreaticoduodenectomy.
[8] The sheet-shaped cell culture according to any one of [1] to [7], in which the sheet-shaped cell culture is used for preventing or treating pancreatic fistula.
[9] The sheet-shaped cell culture according to any one of [1] to [8], further including skeletal myoblast cells.
[10] The sheet-shaped cell culture according to any one of [1] to [9], further including a reinforcement layer containing a gel and/or a polymer.
[11] A method for preventing or treating pancreatic fistula, the method including: a step of covering, using a sheet-shaped cell culture, a cut surface of a pancreas that includes cut surfaces of a pancreatic parenchyma and a pancreatic duct; and a step of connecting the cut surface of the pancreas to an intestinal wall of a small intestine in a liquid-tight manner via the sheet-shaped cell culture.
[12] The method according to [11], further including a step of connecting the pancreatic duct to a through hole in the intestinal wall of the small intestine in a liquid-tight manner via the sheet-shaped cell culture.
[13] The method according to [12], further including a step of, by forming a through hole in the sheet-shaped cell culture at a lumen portion of the cut surface of the pancreatic duct, communicating lumina of the pancreatic duct and the small intestine with each other via the through holes in the sheet-shaped cell culture and the intestinal wall of the small intestine.
[14] The method according to any one of [11] to [13], in which the step of covering, using the sheet-shaped cell culture, the cut surface of the pancreas that includes the cut surfaces of the pancreatic parenchyma and the pancreatic duct is performed by a transplantation device.

### Advantageous Effect of Invention

According to the invention, it is possible to maintain the liquid-tightness of the suture site after the surgical treatment of the pancreas, and it is possible to prevent or treat pancreatic fistula. In particular, according to the invention, it is possible to maintain the liquid-tightness of the suture site after a treatment of pancreaticoduodenectomy, and it is possible to prevent or treat pancreatic fistula. Further, according to the invention, since not only pancreatic fistula can be prevented or treated, but also no adhesion occurs at a site other than the suture site in an abdominal cavity and no inflammation occurs, a prognosis after the surgical treatment of the pancreas can be improved. In addition, according to the invention, by using the sheet-shaped cell culture, it is also possible to promote the fusing of biological tissues at the suture site after the surgical treatment of the pancreas.

### Brief Description of Drawings

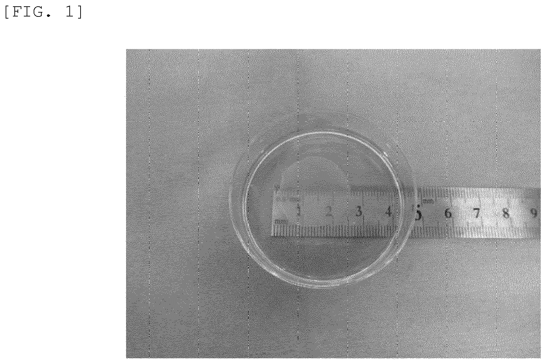

[FIG. 1] FIG. 1 shows a prepared sheet-shaped cell culture.

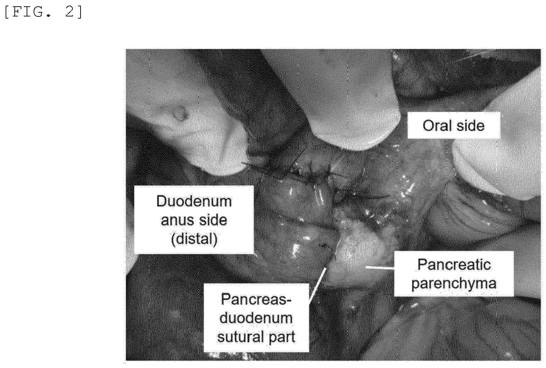

[FIG. 2] FIG. 2 shows a surgical site of a control group for pancreaticoduodenectomy.

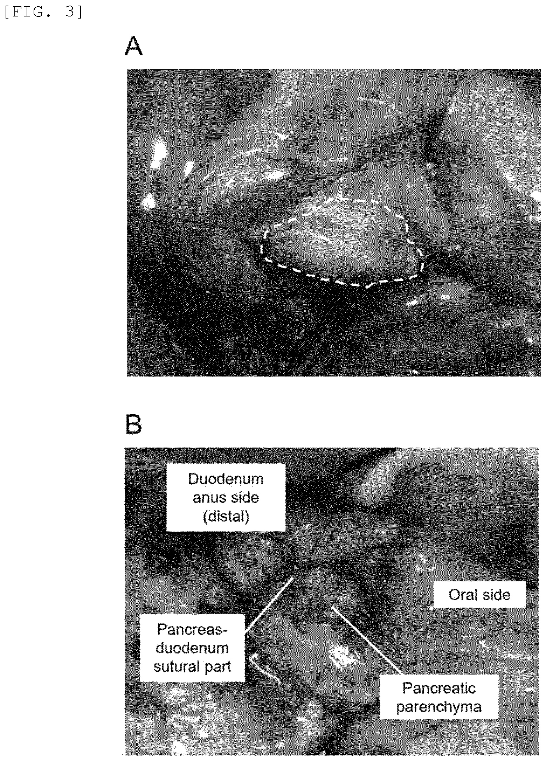

[FIG. 3] FIG. 3 shows a surgical site of a sheet treatment group for pancreaticoduodenectomy. A pancreatic head portion and an anus-side piece of a duodenal papilla portion are subjected to complication ablation, duodenum stumps are anastomosed in an end-to-end manner, and after the sheet-shaped cell culture is transplanted to a pancreatic head incision surface (a region surrounded by a dotted line A in FIG. 3), a distal duodenum and a pancreatic parenchyma are anastomosed (B in FIG. 3).

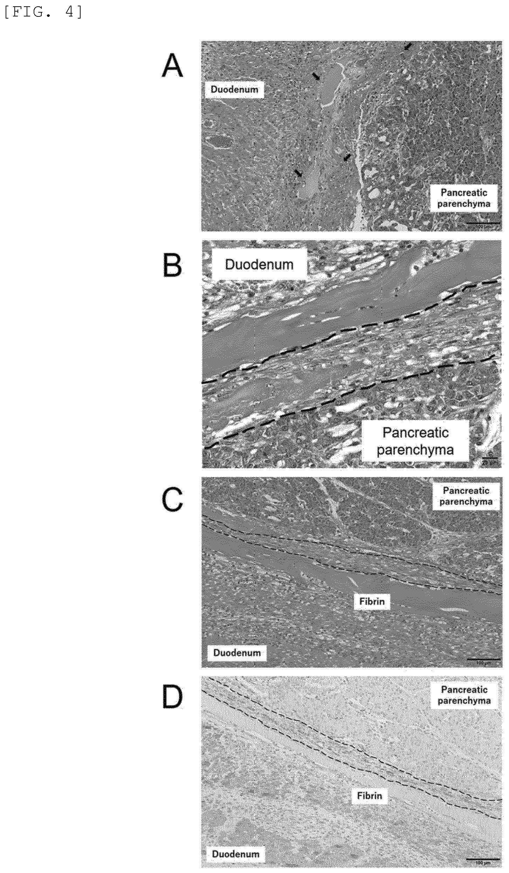

[FIG. 4] A in FIG. 4 is an HE staining image (x200) of an anastomotic site of the duodenum and the pancreatic parenchyma resected 3 days after pancreaticoduodenectomy, of the control group. Arrows indicate hemorrhage and concentrated proteins. B and C in FIG. 4 show HE staining images (x200) of a transplanted site of the sheet-shaped cell culture resected 3 days after the transplantation of the sheet-shaped cell culture, of the sheet treatment group. A region sandwiched by dotted lines indicates the sheet-shaped cell culture. D in FIG. 4 shows an anti-DESMIN antibody immunostaining image (x200) of the transplanted site of the sheet-shaped cell culture resected 3 days after the transplantation of the sheet-shaped cell culture, of the sheet treatment group. A region sandwiched by dotted lines indicates the sheet-shaped cell culture.

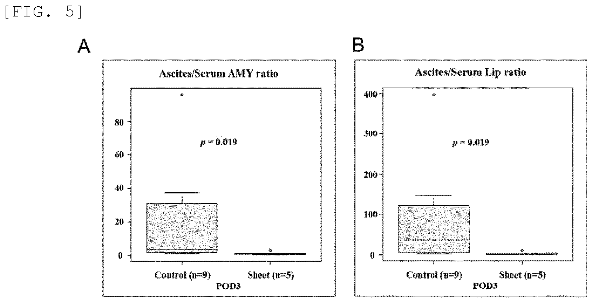

[FIG. 5] FIG. 5 shows (an amylase value of ascites)/ (an amylase value of serum) (A in FIG. 5) and (a lipase value of ascites)/ (a lipase value of serum) (B in FIG. 5) obtained 3 days after pancreaticoduodenectomy. The figures show box plots of the control group (n = 9) and the sheet treatment group (n = 5), a bar at an upper end indicates a maximum value, lines at an upper end, an inner part, and a lower end of a box indicate a third quartile, a second quartile (a median value), and a first quartile, a bar at a lower end indicates a minimum value, and values in the figures indicate p values between the two groups (U test of Mann-Whitney).

Unless otherwise defined in the present description, all technical terms and scientific terms used in the description have the same meanings as terms commonly understood by a person skilled in the art. All patents, applications, and other published materials or information referenced in the description are incorporated herein by reference in their entireties.

### Description of Embodiments

### [Sheet-shaped Cell Culture for Covering Cut Surface of Pancreas]

One aspect of the invention relates to a sheet-shaped cell culture for covering a cut surface of a pancreas, the cut surface of the pancreas includes cut surfaces of a pancreatic parenchyma and a pancreatic duct, and is connected to an intestinal wall of a small intestine in a liquid-tight manner via the sheet-shaped cell culture (sometimes referred to as "the sheet-shaped cell culture according to the invention").

In an embodiment, the pancreatic duct is connected to a through hole in the intestinal wall of the small intestine in a liquid-tight manner via the sheet-shaped cell culture.

In an embodiment, by forming a through hole in the sheet-shaped cell culture at a lumen portion of the cut surface of the pancreatic duct, lumina of the pancreatic duct and the small intestine communicate with each other via the through holes in the sheet-shaped cell culture and the intestinal wall of the small intestine.

In an embodiment, the pancreatic duct is a main pancreatic duct and/or an accessory pancreatic duct. In an embodiment, the small intestine is a duodenum or a jejunum.

In an embodiment, the cut surface of the pancreas, the through hole in the intestinal wall of the small intestine, and/or the through hole of the sheet-shaped cell culture is formed by a surgical treatment.

In an embodiment, the surgical treatment is pancreaticoduodenectomy.

In an embodiment, the sheet-shaped cell culture according to the invention is used to prevent or treat pancreatic fistula.

In an embodiment, the sheet-shaped cell culture according to the invention includes skeletal myoblast cells.

In an embodiment, the sheet-shaped cell culture according to the invention includes a reinforcement layer containing a gel and/or a polymer.

The pancreas is an exocrine gland that secretes pancreatic juice and delivers the pancreatic juice into a duodenum. A duodenum-side site of the pancreas is referred to as a pancreatic head portion, a spleen-side site thereof is referred to as a pancreatic tail portion, and a site between the pancreatic head portion and the pancreatic tail portion is referred to as a pancreatic body portion. The pancreas has pancreatic ducts in which the pancreatic juice is carried to the duodenum. A large number of branched thin pancreatic ducts merge from the pancreatic tail portion to the pancreatic head portion to form a main pancreatic duct (and an accessory pancreatic duct), and are connected to the duodenum. In the case of humans, before being connected to the duodenum, the main pancreatic duct merges with a common bile duct carrying out bile from a gallbladder. The pancreatic ducts are connected to an intestinal wall of the duodenum, and discharge the pancreatic juice to a lumen of the duodenum. An opening of the pancreatic ducts rises toward the lumen of the duodenum, and is called a duodenal papilla.

In the invention, the pancreas may be a pancreas of any organism, and is, for example, a pancreas from humans, non-human primates, rodents (mice, rats, hamsters, guinea pigs, or the like), and mammals such as rabbits, dogs, cats, pigs, horses, cows, goats, and sheep, but is not limited thereto.

Further, the pancreas can be said to be an organ that has lumina such as a pancreatic duct, a main pancreatic duct, an accessory pancreatic duct, and the like therein. A biological fluid such as digestive juice flows through the lumina. According to the invention, the cut surface of the pancreas is connected to the intestinal wall of the small intestine in a liquid-tight manner via the sheet-shaped cell culture, and the pancreatic duct is connected to the through hole in the intestinal wall of the small intestine in a liquid-tight manner via the sheet-shaped cell culture, whereby a liquid-tightness of a suture site of the lumina after the surgical treatment of the pancreas can be maintained. Accordingly, it is possible to prevent or treat the leakage of the biological liquid such as digestive juice flowing through the lumina from the suture site to the outside of the pancreas or the small intestine. In particular, according to the invention, it is possible to maintain the liquid-tightness of the suture site of the lumina after the treatment of pancreaticoduodenectomy. Accordingly, it is possible to prevent or treat the leakage of the biological liquid such as digestive juice flowing through the lumina from the suture site to the outside of the pancreas or the small intestine.

In the invention, the phrase "cut surface of the pancreas" refers to a cross section and/or a cross section of a fragment obtained as a result of making an incision on the pancreas at any site and/or cutting the pancreas off at any site. The cut surface of the pancreas is, for example, a cross section of a fragment generated as a result of cutting the pancreas off between the pancreatic head portion and the pancreatic body portion. In an embodiment, the cut surface of the pancreas includes the cut surfaces of the pancreatic parenchyma and the pancreatic duct, and here, the cut surface of the pancreatic duct is, for example, a cut surface of the main pancreatic duct and/or the accessory pancreatic duct.

In the invention, the phrase "covering a cut surface of a pancreas" refers to covering all of the cut surface of the pancreas by the sheet-shaped cell culture. A size of the sheet-shaped cell culture is equal to or more than that of the cut surface of the pancreas, and may exactly cover all of the cut surface of the pancreas, or may cover all of the cut surface of the pancreas and have an extra edge portion. The edge portion may further cover a vicinity of the cut surface of the pancreas.

In the invention, the sheet-shaped cell culture may have a through hole, and "covering a cut surface of a pancreas" includes covering all of the cut surface of the pancreas by a sheet-shaped cell culture having a through hole at any position. At this time, the sheet-shaped cell culture is disposed such that the through hole thereof is aligned with a position of a lumen portion of the cut surface of the pancreatic duct.

In the invention, the "sheet-shaped cell culture" means a cell culture formed into a sheet shape by connecting cells to each other. The cells may be connected to each other directly (including a case in which the cells are connected via a cell element such as an adhesion molecule) and/or via an intervening substance. The intervening substance is not particularly limited as long as the intervening substance is a substance that can connect cells to each other at least physically (mechanically), and examples thereof include extracellular matrices. The intervening substance is one that is preferably derived from cells, and is one that is particularly derived from cells constituting a cell culture. The cells are at least physically (mechanically) connected, and may further be functionally connected, for example, chemically or electrically connected. The sheet-shaped cell culture may be composed of one cell layer (a single layer), or may be composed of two or more cell layers (a laminate (a multilayer) of, for example, two layers, three layers, four layers, five layers, six layers, and the like). Further, the sheet-shaped cell culture may have a three-dimensional structure which has a thickness exceeding a thickness of one cell without the cells showing a clearly layered structure. For example, in a vertical cross section of the sheet-shaped cell culture, the cells may be present in a non-uniformly disposed state (for example, a mosaic-like state) without being uniformly aligned in a horizontal direction.

The sheet-shaped cell culture preferably does not contain a scaffold (a support). A scaffold is used sometimes in the related art to allow cells to adhere onto a surface and/or to adhere inside of the scaffold, thereby maintaining physical integrity of a sheet-shaped cell culture, for example, films made of polyvinylidene difluoride (PVDF) and the like are known, but the sheet-shaped cell culture according to the invention can maintain the physical integrity without the scaffold. In addition, the sheet-shaped cell culture according to the invention is preferably composed of only a substance derived from the cells constituting the sheet-shaped cell culture, and does not contain any other substance.

The cells constituting the sheet-shaped cell culture are not particularly limited as long as the cells can form the sheet-shaped cell culture, and examples thereof include adherent cells (adhesive cells). Examples of the adherent cells include adherent somatic cells (for example, cardiomyocytes, fibroblast cells, epithelial cells, endothelial cells, hepatocytes, pancreatic cells, renal cells, adrenal cells, periodontal ligament cells, gingival cells, periosteal cells, skin cells, synovial cells, and chondrocytes) and stem cells (tissue stem cells such as myoblast cells and cardiac stem cells, pluripotent stem cells such as embryonic stem cells and induced pluripotent stem (iPS) cells, mesenchymal stem cells). Somatic cells may be differentiated from stem cells, particularly from iPS cells (adherent cells derived from iPS cells). Nonlimiting examples of the cells constituting the sheet-shaped cell culture include myoblast cells (for example, skeletal myoblast cells), mesenchymal stem cells (for example, cells derived from bone marrow, adipose tissue, peripheral blood, skin, hair root, muscle tissue, endometrium, placenta, or cord blood), cardiomyocytes, fibroblast cells, cardiac stem cells, embryonic stem cells, iPS cells, synovial cells, chondrocytes, epithelial cells (for example, oral mucosal epithelial cells, retinal pigment epithelial cells, nasal mucosal epithelial cells), endothelial cells (for example, vascular endothelial cells), hepatocytes (for example, hepatic parenchymal cells), pancreatic cells (for example, pancreatic islet cells), renal cells, adrenal cells, periodontal ligament cells, gingival cells, periosteal cells, and skin cells. Nonlimiting examples of adherent cells derived from iPS cells include cardiomyocytes, fibroblast cells, epithelial cells, endothelial cells, hepatocytes, pancreatic cells, renal cells, adrenal cells, periodontal ligament cells, gingival cells, periosteal cells, skin cells, synovial cells, and chondrocytes, all of which are derived from iPS cells.

In the invention, the term "skeletal myoblast cells" means myoblast cells present in skeletal muscle. Skeletal myoblast cells can be prepared from skeletal muscle by any known method in the related art (for example, the method described in JP2007-89442A), and are commercially available (for example, Lonza, Cat# CC-2580). Skeletal myoblast cells can be identified by, for example, markers such as CD56, α7 integrin, myosin heavy chain IIa, myosin heavy chain IIb, myosin heavy chain IId (IIx), MyoD, Myf5, Myf6, myogenin, desmin, and PAX3, but are not limited thereto. In a specific embodiment, skeletal myoblast cells are CD56-positive. In a further specific embodiment, skeletal myoblast cells are CD56-positive and desmin-positive. Skeletal myoblast cells are derived from any organism having skeletal muscle, for example, skeletal myoblast cells may be derived from humans, non-human primates, rodents (mice, rats, hamsters, guinea pigs, or the like), and mammals such as rabbits, dogs, cats, pigs, horses, cows, goats, and sheep, but are not limited thereto. In an embodiment, skeletal myoblast cells are skeletal myoblast cells of mammals. In a specific embodiment, skeletal myoblast cells are human skeletal myoblast cells. Further, skeletal myoblast cells can be collected from any skeletal muscle. In an embodiment, the skeletal myoblast cells according to the invention are skeletal myoblast cells derived from a femoral region, a neck region, or an abdominal region.

The cells constituting the sheet-shaped cell culture may be derived from any organism that can be treated with the sheet-shaped cell culture. Examples of the organism include humans, non-human primates, dogs, cats, pigs, horses, goats, sheep, rodents (for example, mice, rats, hamsters, and guinea pigs), and rabbits, but are not limited thereto. Further, the number of types of cells constituting the sheet-shaped cell culture is not particularly limited, and the sheet-shaped cell culture may be constituted by only one type of cells, or by two or more types of cells. When there are two or more types of cells forming the sheet-shaped cell culture, a content ratio (degree of purity) of the most abundant cells is 50% or more, preferably 60% or more, more preferably 70% or more, and even more preferably 75% or more at the point in which formation of the sheet-shaped cell culture is completed.

The cells may be xenogeneic cells or allogeneic cells. Here, the term "xenogeneic cells" refers to herein means cells derived from an organism of a species different from that of a recipient of the xenogeneic cells when the sheet-shaped cell culture is used for transplantation. For example, cells derived from monkeys or pigs and the like correspond to the xenogeneic cells when the recipient is human. Further, the term "allogeneic cells" means cells derived from an organism of the same species as that of the recipient. For example, human cells correspond to the allogeneic cells when the recipient is human. Allogeneic cells include cells of autologous origin (also called autologous cells), that is, cells derived from a recipient, and allogeneic non-autologous cells (also called allogeneic cells). Autologous cells are preferable in the invention because autologous cells do not cause a rejection reaction when transplanted. However, it is also possible to use xenogeneic cells or allogeneic non-autologous cells. When xenogeneic cells or allogeneic non-autologous cells are used, an immunosuppressive treatment may be required to suppress a rejection reaction. Further, in the description, cells other than autologous cells, that is, xenogeneic cells and allogeneic non-autologous cells may be referred to as non-autologous cells. In an embodiment of the invention, cells are autologous cells or allogeneic cells. In an embodiment of the invention, cells are autologous cells. In another embodiment of the invention, cells are allogeneic cells.

The sheet-shaped cell culture can be produced by any known method (for example, see PTL 1, JP2010-081829A, and JP2011-110368A). A method for producing a sheet-shaped cell culture typically includes a step of seeding cells on a culture substrate, a step of forming the seeded cells into a sheet, and a step of detaching the formed sheet-shaped cell culture from the culture substrate, but is not limited thereto. Before the step of seeding the cells on the culture substrate, a step of freezing the cells and a step of thawing the cells may be performed. Further, a step of washing the cells may be performed after the step of thawing the cells. Each of these steps can be performed by any known method suitable for producing the sheet-shaped cell culture. The method for producing a sheet-shaped cell culture may include a step of producing a sheet-shaped cell culture, and in this case, the step of producing a sheet-shaped cell culture may include one or two or more steps relating to the method for producing a sheet-shaped cell culture described above as sub-steps. In an embodiment, a step of proliferating the cells is not included after the step of thawing the cells and before the step of seeding the cells on the culture substrate.

The culture substrate is not particularly limited as long as cells can form a cell culture thereon, and examples thereof include containers made of various materials, and solid or semi-solid surfaces of the containers. A container preferably has a structure or material which does not allow liquids such as a culture solution to permeate. Examples of the material include polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, and metals (for example, iron, stainless steel, aluminum, copper, brass), but are not limited thereto. In addition, the container preferably has at least one flat surface. Examples of the container include a culture container having a bottom surface composed of a culture substrate on which a cell culture can be formed, and a liquid-impermeable side surface, but are not limited thereto. Specific examples of the culture container include cell culture dishes, and cell culture bottles, but are not limited to. The bottom surface of the container may be transparent or opaque. When the bottom surface of the container is transparent, cells can be observed, counted, and the like from a back side of the container. Further, the container may have a solid or semi-solid surface therein. Examples of the solid surface include plates and containers made of various materials as described above, and examples of the semi-solid surface include gels and soft polymer matrices. The culture substrate may be produced using the materials described above, or may use commercially available products. Preferable examples of the culture substrate include a substrate having an adhesive surface suitable for forming a sheet-shaped cell culture, but are not limited thereto. Specific examples thereof include a substrate having a hydrophilic surface, for example, a substrate having a surface coated with hydrophilic compounds such as polystyrene subjected to a corona discharge treatment, collagen gels, and hydrophilic polymers, and a substrate having a surface coated with extracellular matrices such as collagen, fibronectin, laminin, vitronectin, proteoglycan, and glycosaminoglycan, and cell adhesion factors such as the cadherin family, the selectin family, and the integrin family. Further, the substrates are commercially available (for example, Corning (registered trademark) TC-Treated Culture Dish, Corning). All or a part of the culture substrate may be transparent or opaque.

A surface of the culture substrate may be coated with a material having physical properties that change in response to stimuli such as temperature and light. As the materials, it is possible to use known materials such as a temperature-responsive material consisting of a homopolymer or a copolymer of a (meth)acrylamide compound, N-alkyl substituted (meth)acrylamide derivatives (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, and N-tetrahydrofurfurylmethacrylamide), N,N-dialkyl substituted (meth)acrylamide derivatives (for example, N,N-dimethyl(meth)acrylamide, N,N-ethylmethylacrylamide, and N,N-diethylacrylamide), (meth)acrylamide derivatives having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, and 4-(1-oxo-2-methyl-2-propenyl)-morpholine), or vinyl ether derivatives (for example, methyl vinyl ether), and a photoresponsive material such as a light absorbing polymer having an azobenzene group, a copolymer of a vinyl derivative of triphenylmethane leuco hydroxide and an acrylamide-based monomer, and N-isopropylacrylamide gel containing spirobenzopyran (see, for example, JPH02-211865A and JP2003-33177A), but is not limited thereto. It is possible to change physical properties such as hydrophilicity and hydrophobicity by imparting a predetermined stimulus to these materials, whereby it is possible to promote detachment of a cell culture adhered onto the same materials. Culture dishes coated with temperature-responsive materials are commercially available (for example, UpCell (registered trademark) from CellSeed Inc.), and the culture dishes can be used in the production method of the sheet-shaped cell culture.

The culture substrate may have various shapes, but is preferably flat. In addition, an area thereof is not particularly limited, and may be, for example, about 1 cm² to about 200 cm², about 2 cm² to about 100 cm², about 3 cm² to about 50 cm². For example, a circular culture dish having a diameter of 10 cm may be used as the culture substrate. In this case, an area thereof is 56.7 cm².

The culture substrate may be coated with serum. By using the culture substrate coated with serum, it is possible to form a sheet-shaped cell culture with a higher density. The phrase "coated with serum" means a state where serum components are adhered to a surface of the culture substrate. The state can be obtained by, for example, treating the culture substrate with serum, but is not limited thereto. The treatment with serum includes bringing serum into contact with the culture substrate and optionally incubating the culture substrate for a predetermined time.

Xenogeneic serum and/or allogeneic serum can be used as the serum. The term xenogeneic serum means serum derived from an organism of a species different from that of a recipient when the sheet-shaped cell culture is used for transplantation. For example, when the recipient is human, serum derived from cows or horses, such as fetal bovine serum (FBS, FCS), calf serum (CS), and horse serum (HS) corresponds to the xenogeneic serum. In addition, the term "allogeneic serum" means serum derived from an organism of the same species as that of the recipient. For example, when the recipient is human, human serum corresponds to the allogeneic serum. Allogeneic serum includes autologous serum (also called autologous serum), that is, serum derived from a recipient, and allogeneic non-autologous serum derived from an allogeneic individual other than the recipient. In the description, sera other than autologous serum, that is, xenogeneic serum and allogeneic non-autologous serum may be referred to as non-autologous serum.

Serum for coating the culture substrate is commercially available, or can be prepared by a common method from blood collected from a desired organism. Specifically, examples of the method includes a method in which collected blood is allowed to stand at room temperature for about 20 minutes to about 60 minutes to coagulate, the coagulated blood is centrifuged at about 1000×g to about 1200×g, and a supernatant is collected.

In a case of incubation on the culture substrate, serum may be used as a stock solution or may be diluted and used. Dilution can be performed with any medium such as water, a physiological salt solution, various buffer solutions (for example, PBS, HBSS), and various liquid media (for example, DMEM, MEM, F12, DMEM/F12, DME, RPMI1640, MCDB (MCDB102, 104, 107, 120, 131, 153, 199, or the like), L15, SkBM, RITC80-7), but examples of the medium are not limited thereto. A dilution concentration is not particularly limited as long as the serum components can adhere onto the culture substrate, and for example, is about 0.5% to about 100% (v/v), preferably about 1% to about 60% (v/v), and more preferably about 5% to about 40% (v/v).

An incubation time is also not particularly limited as long as the serum components can adhere onto the culture substrate, and for example, is about 1 hour to about 72 hours, preferably about 2 hours to about 48 hours, more preferably about 2 hours to about 24 hours, and even more preferably about 2 hours to about 12 hours. An incubation temperature is also not particularly limited as long as the serum components can adhere onto the culture substrate, and for example, is about 0°C to about 60°C, preferably about 4°C to about 45°C, and more preferably a room temperature to about 40°C.

Serum may be discarded after incubation. As a method for discarding serum, a commonly used liquid discarding method such as suction with a pipette or decantation can be used. In a preferred embodiment of the invention, the culture substrate may be washed with a serum-free washing solution after discarding serum. The serum-free washing solution is not particularly limited as long as the serum-free washing solution is a liquid medium not containing serum and not adversely affecting serum components adhered to the culture substrate. Washing can be performed using water, a physiological salt solution, various buffer solutions (for example, PBS, HBSS, and the like), and various liquid media (for example, DMEM, MEM, F12, DMEM/F12, DME, RPMI1640, MCDB (MCDB102, 104, 107, 120, 131, 153, 199, and the like), L15, SkBM, RITC80-7), but is not limited thereto. As a washing method, it is possible to use a commonly used culture substrate washing method such as a method in which a serum-free washing solution is added onto a culture substrate, stirred for a predetermined time (for example, about 5 seconds to about 60 seconds), and then discarded, but the washing method is not limited thereto.

In the invention, the culture substrate may be coated with a growth factor. The term "growth factor" referred to herein means any substance promoting proliferation of cells as compared to a case where a substance promoting proliferation of cells does not exist, and examples thereof include an epidermal growth factor (EGF), a vascular endothelial growth factor (VEGF), and a fibroblast growth factor (FGF). A method for coating a culture substrate with a growth factor, a method for discarding a growth factor, and a method for washing a culture substrate are basically the same methods as those for serum except that a dilution concentration at the time of incubation is, for example, about 0.0001 µg/mL to about 1 µg/mL, preferably about 0.0005 µg/mL to about 0.05 µg/mL, and more preferably about 0.001 µg/mL to about 0.01 µg/m L.

In the invention, the culture substrate may be coated with a steroid drug. Here, the term "steroid drug" refers to a compound that can exert an adverse effect on a living body such as adrenocortical hypofunction and Cushing syndrome among compounds having a steroid nucleus. Examples of the compound include cortisol, prednisolone, triamcinolone, dexamethasone, and betamethasone, but are not limited thereto. A method for coating a culture substrate with a steroid drug, a method for discarding a steroid drug, and a method for washing a culture substrate are basically the same methods as those for serum except that a dilution concentration at the time of incubation is, for example, about 0.1 µg/mL to about 100 µg/mL, preferably about 0.4 µg/mL to about 40 µg/mL, more preferably about 1 µg/mL to about 10 ug/mL when the steroid drug is dexamethasone.

The culture substrate may be coated with any one of serum, a growth factor, and a steroid drug, or may be coated with any combination thereof, that is, a combination of serum and a growth factor, serum and a steroid drug, serum, a growth factor and a steroid drug, or a growth factor and a steroid drug. In a case of coating with multiple components, these components may be mixed to be applied at the same time, or may be applied in separate steps.

The culture substrate may be seeded with cells immediately after being coated with serum or the like, or may be stored in advance after being coated and then seeded with cells. The coated substrate can be stored for a long period of time by keeping the coated substrate at, for example, about 4°C or lower, preferably about -20°C or lower, and more preferably about -80°C or lower.

Seeding of cells on the culture substrate can be performed by any known method and condition. The seeding of cells on the culture substrate may be performed by, for example, injecting a cell suspension obtained by cells being suspended in a culture solution into the culture substrate (a culture container). An instrument suitable for an injection operation of a cell suspension, such as a dropper and a pipette, can be used for injecting the cell suspension.

The seeding can be performed at a density of about 7.1×10⁵ cells/cm² to about 3.0×10⁶ cells/cm², about 7.3×10⁵ cells/cm² to about 2.8×10⁶ cells/cm², about 7.5×10⁵ cells/cm² to about 2.5×10⁶ cells/cm², about 7.8×10⁵ cells/cm² to about 2.3×10⁶ cells/cm², about 8.0×10⁵ cells/cm² to about 2.0×10⁶ cells/cm², about 8.5×10⁵ cells/cm² to about 1.8×10⁶ cells/cm², about 9.0×10⁵ cells/cm² to about 1.6×10⁶ cells/cm², about 1.0×10⁶ cells/cm² to about 1.6×10⁶ cells/cm², and the like.

The cells constituting the sheet-shaped cell culture according to the invention (sometimes referred to as "sheet-forming cells") are as described above in detail. The sheet-shaped cell culture according to the invention is not required to contain cells of an application tissue, but the sheet-shaped cell culture rather may contain cells that are not present in the tissue. Accordingly, in an embodiment, the sheet-shaped cell culture according to the invention contains ectopic cells, that is, cells that are not originally present in an application tissue. In a preferred embodiment, as described above, the sheet-shaped cell culture according to the invention is applied for damage of tissues of a pancreas and a small intestine, and examples of ectopic cells in this case include cells derived from striated muscle such as skeletal myoblast cells, and mesenchymal stem cells. In a further preferred embodiment, the sheet-shaped cell culture according to the invention contains skeletal myoblast cells. The skeletal myoblast cells may be derived from striated muscle of a femoral region, a neck region, or an abdominal region.

When skeletal myoblast cells are prepared from a striated muscle tissue, a prepared cell population contains fibroblast cells. When the cell population containing skeletal myoblast cells prepared from the striated muscle tissue is used in the production of the sheet-shaped cell culture according to the invention, the cell population contains a certain amount of fibroblast cells. Fibroblast cells are well known in the related art, and can be identified by markers such as TE-7 (see, for example, Rosendaal et al., J Cell Sci. 1994; 107 (Pt 1): 29-37, Goodpaster et al., J Histochem Cytochem. 2008; 56(4): 347-58) .

In an embodiment, the cells forming the sheet-shaped cell culture according to the invention include skeletal myoblast cells prepared from a striated muscle tissue. Accordingly, a cell population to be used in the production of the sheet-shaped cell culture according to the invention may contain skeletal myoblast cells and fibroblast cells. In an embodiment, a CD56-positive rate of a cell population used in the production of the sheet-shaped cell culture according to the invention may be 50% or more, 60% or more, 65% or more, 70% or more, 75% or more, 80% or more, 85% or more, and 90% or more, and is preferably 60% or more.

The cell population used in the production of the sheet-shaped cell culture according to the invention may contain fibroblast cells, but when a content of fibroblast cells is too high, the cell population is not preferable because a content of skeletal myoblast cells decreases. Accordingly, in an embodiment, a TE7-positive rate of a cell population used in the production of the sheet-shaped cell culture according to the invention may be 50% or less, 40% or less, 35% or less, 30% or less, 25% or less, 20% or less, 150 or less, and 10% or less, and is preferably 40% or less.

The cell population used in the production of the sheet-shaped cell culture according to the invention may contain cells other than skeletal myoblast cells and fibroblast cells, but it is preferable that the number of such cells is as small as possible. Accordingly, a total value of the CD56-positive rate and the TE7-positive rate is preferably high, and for example, may be 80% or more, 85% or more, 90% or more, 95% or more, 96% or more, 97% or more, 98% or more, 99% or more or the like, and is preferably 90% or more.

A thickness of the sheet-shaped cell culture according to the invention is not particularly limited. When a single layer sheet is used as the sheet-shaped cell culture, a thickness thereof is generally a thickness of one cell or more, and the thickness varies depending on the type of sheet-forming cells. In an embodiment, a thickness of the sheet-shaped cell culture according to the invention is 30 um or more, and in a preferred embodiment, the thickness is 50 µm or more. A thickness range of the sheet-shaped cell culture according to the invention is, for example, 30 um to 200 um, preferably 50 um to 150 µm, and more preferably 60 um to 100 um. When a laminated sheet is used as the sheet-shaped cell culture, a thickness thereof is set such that the thickness does not exceed a thickness of the single layer sheet × the number of laminated layers. Accordingly, for example, when a sheet in which five single layer sheets are laminated is used as an embodiment, a thickness thereof is 150 um or more, preferably 250 um or more, and a thickness range is, for example, 150 um to 1000 um, preferably 250 um to 750 µm, and more preferably 300 µm to 500 um.

Accordingly, the thickness of the sheet-shaped cell culture according to the invention is, for example, 30 um to 1000 µm, and preferably 50 µm to 750 um, 50 um to 500 µm, and 60 µm to 500 µm.

In a certain embodiment, the sheet-shaped cell culture according to the invention may be very fragile and difficult to handle. Accordingly, the sheet-shaped cell culture according to the invention may further have a reinforcement layer for the purpose of facilitating the handling and reducing the risk of breakage. The reinforcement layer may be any layer as long as the layer can reinforce a structure without impairing a function of the sheet-shaped cell culture according to the invention, and the reinforcement layer may be a reinforcement layer containing, for example, a gel and/or a polymer, but since the reinforcement layer is to be transplanted into a living body, the reinforcement layer is preferably a biocompatible reinforcement layer containing, for example, a biocompatible gel or polymer.

The gel, preferably a biocompatible gel that can be used for the reinforcement layer of the invention may be any gel as long as the gel does not adversely affect a living body when the gel is introduced into the living body, and examples thereof include a fibrin gel, a fibrinogen gel, a gelatin gel, and a collagen gel, but are not limited thereto.

The polymer, preferably a biocompatible polymer that can be used in the reinforcement layer of the invention may be any polymer as long as the polymer does not adversely affect a living body when the polymer is introduced into the living body, and examples thereof include polylactic acid, polydioxano, polyglycapro, and collagen, but are not limited thereto.

As a method for forming a reinforcement layer containing a biocompatible gel, methods known in the related art can be used. Examples of the methods include a method for spraying a biocompatible gel or polymer, or a component serving as a material thereof on a sheet-shaped cell culture, a method for laminating a sol-like biocompatible substance on a sheet-shaped cell culture and gelling the same, a method for immersing a sheet-shaped cell culture in a liquid gel and then solidifying the gel, and the method disclosed in JP2016-52271A, but are not limited thereto.

Since the reinforcement layer is used for facilitating the handling of the sheet-shaped cell culture according to the invention and reducing the risk of breakage, the reinforcement layer preferably has a certain strength or higher, and preferably has elasticity. A known evaluation unit for strength of a structure containing a gel or polymer is, for example, a jelly strength, and a known evaluation unit for strength of a sheet-shaped structure is, for example, a tensile breaking load. A method for measuring a jelly strength is described in, for example, JIS K 6503. The tensile breaking load means a maximum load until both ends of a sheet-shaped cell culture or the like are pulled in a horizontal direction to break, and a measuring method thereof is described in, for example, JP2016-52272A.

A tensile breaking load of the reinforcement layer of the sheet-shaped cell culture according to the invention may be about 0.010N or more, about 0.015N or more, about 0.020N or more, about 0.025N or more, about 0.030N or more, about 0.035N or more, about 0.040N or more, or about 0.045N or more, but is not limited thereto, or the tensile breaking load may be within a range of about 0.010N to about 0.200N, about 0.015N to about 0.100N, or about 0.020N to about 0.50N. As compared to a sheet-shaped cell culture not having the reinforcement layer, a strength of the sheet-shaped cell culture having the reinforcement layer may be about 1.5 times or more, about 2 times or more, about 3 times or more, about 4 times or more, about 5 times or more, about 6 times or more, about 7 times or more, about 8 times or more, about 9 times or more, and about 10 times or more, or the strength may be within a range of about 1.5 times to about 20 times, about 2 times to about 15 times, or about 2.5 times to about 10 times.

When the sheet-shaped cell culture having the reinforcement layer is applied, the sheet-shaped cell culture is preferably applied such that the reinforcement layer does not directly come into contact with an application site. That is, the sheet-shaped cell culture is preferably applied such that the sheet-shaped cell culture is located between the application site and the reinforcement layer.

The application of the sheet-shaped cell culture to the application site can be performed using any device and/or method known in the related art.

In an embodiment, when the sheet-shaped cell culture according to the invention is applied to a tissue, the sheet-shaped cell culture may be applied in combination with other compositions and/or grafts which promote healing or fusing. Examples of other compositions and/or grafts which promote healing or fusing include grafts containing pedunculated blood vessels such as pedunculated omentum grafts, fibrin gels, and Ad-spray (registered trademark), but are not limited thereto. In a preferred embodiment, the sheet-shaped cell culture according to the invention is applied together with a graft containing pedunculated blood vessels. Typical examples of the graft containing pedunculated blood vessels include, for example, pedunculated omentum grafts.

Such other compositions and/or grafts which promote healing or fusing may be compositions or grafts independent from the sheet-shaped cell culture according to the invention, and such other compositions or grafts may be substances incorporated in, for example, a sheet-shaped cell culture or a reinforcement layer.

When the sheet-shaped cell culture according to the invention is applied together with independent other compositions and/or grafts which promote healing or fusing, such other compositions and/or grafts may be applied before the application of the sheet-shaped cell culture or may be applied after the application. When such other compositions and/or grafts are applied before the application of the sheet-shaped cell culture, such other compositions and/or grafts are applied to be located between the application site and the sheet-shaped cell culture. That is, first, such other compositions and/or grafts are applied to the application site, and then the sheet-shaped cell culture (optionally containing the reinforcement layer) is applied thereon. When such other compositions and/or grafts are applied after the application of the sheet-shaped cell culture, such other compositions and/or grafts are applied to the application site from above the sheet-shaped cell culture (optionally containing the reinforcement layer). That is, first, the sheet-shaped cell culture is applied to the application site, and then such other compositions and/or grafts are applied thereon.

As an tissue regeneration mechanism, there is a mechanism by a paracrine effect in which a sheet-shaped cell culture continuously releases cytokines such as VEGF and HGF, and collagen, which are responsible for, for example, angiogenesis, cell protection, repair, and the like at a damaged site, and/or a mechanism by an autocrine effect in which collagen production and the like are promoted by activating progenitor cells and stem cells of a surrounding tissue to which the sheet-shaped cell culture is applied.

A size of the sheet-shaped cell culture according to the invention is not particularly limited as long as the sheet-shaped cell culture can cover a predetermined site of a biological tissue, and for example, when a shape of the sheet-shaped cell culture is circular, a diameter thereof is 10 mm to 55 mm, 15 mm to 50 mm, 20 mm to 45 mm, 25 mm to 40 mm, or 30 mm to 35 mm, and is preferably 30 mm to 35 mm from the viewpoint of covering the cut surface of the pancreas.

The small intestine is a digestive tract following a stomach and connected to a large intestine. A stomach-side site is called duodenum, a large intestine-side site is called ileum, and a site between the duodenum and the ileum is called jejunum. The duodenum surrounds the pancreatic head portion of the pancreas while being curved in a C shape. The opening of the pancreatic ducts of the pancreas rises toward the lumen of the duodenum, and is called a duodenal papilla. The pancreatic juice and the bile are supplied from the opening.

In the invention, the term "intestinal wall of the small intestine" means a wall at any site of the small intestine that partitions the inside and the outside of an intestinal tract of the small intestine. The intestinal wall of the small intestine is composed of a serous membrane, an outer longitudinal muscle layer, an inner circular muscle layer, a submucosal tissue, a muscularis mucosae, a lamina propria, a mucosal epithelium, and the like. In an embodiment, the intestinal wall of the small intestine is an intestine wall of the duodenum, an intestinal wall of the jejunum, or an intestinal wall of the ileum.

In the invention, the phrase "connected in a liquid-tight manner" means that sites of different biological tissues are connected in close contact with one another so as to prevent the leakage of the biological fluid such as a digestive juice in a biological tissue. In the invention, the term "liquid-tightness" means a property that sites of different biological tissues are connected in a liquid-tight manner. The connecting may be performed using any medical instrument or medical device such as a suture thread, a medical stapler or a suture adhesive, and from the viewpoint of liquid-tightness, the connecting is preferably performed using a suture thread. For example, the sites of different biological tissues are sutured using a suture thread at a portion, an edge portion of a surface, or the like where the sites are connected, and are connected in close contact with one another so as to prevent the leakage of the biological fluid such as a digestive juice in a biological tissue.

In the invention, the phrase "connected in a liquid-tight manner via a sheet-shaped cell culture" means that the sites of different biological tissues are connected in close contact with one another so as to prevent the leakage of the biological fluid such as a digestive juice in a biological tissue in a state where the sheet-shaped cell culture is interposed. That is, due to the connecting, a structure is provided in which "the site of biological tissue - the sheet-shaped cell culture - the site of biological tissue" are connected in close contact with one another in this order. At this time, all of a portion or a surface at a site of one biological tissue connected to another site are covered with the sheet-shaped cell culture. Further, the connecting may be performed using any medical instrument or medical device such as a suture thread, a medical stapler or a suture adhesive, and from the viewpoint of liquid-tightness, the connecting is preferably performed using a suture thread. For example, the sites of different biological tissues are sutured using a suture thread at the portion, the edge portion of the surface, or the like where the sites are connected together with the sheet-shaped cell culture, and are connected in close contact with one another so as to prevent leakage of the biological fluid such as a digestive juice in a biological tissue in the state where the sheet-shaped cell culture is interposed.

In the invention, the phrase "the cut surface of the pancreas is connected to the intestinal wall of the small intestine in a liquid-tight manner via the sheet-shaped cell culture" means that the cut surface of the pancreas and the intestinal wall of the small intestine are connected in close contact with each other so as to prevent the leakage of the biological fluid such as a digestive juice in the pancreas and the small intestine in the state where the sheet-shaped cell culture is interposed. That is, due to the connecting, a structure is provided in which "the cut surface of the pancreas - the sheet-shaped cell culture - the intestinal wall of the small intestine" are connected in close contact with one another in this order. At this time, all of the cut surface of the pancreas is covered with the sheet-shaped cell culture. Further, the connecting may be performed using any medical instrument or medical device such as a suture thread, a medical stapler or a suture adhesive, and from the viewpoint of liquid-tightness, the connecting is preferably performed using a suture thread. For example, the cut surface of the pancreas and the intestinal wall of the small intestine are sutured together with the sheet-shaped cell culture using a suture thread at the portion, the edge portion of the surface, or the like where the cut surface and the intestinal wall are connected, and are connected in close contact with each other so as to prevent the leakage of the biological fluid such as a digestive juice in the pancreas and the small intestine in the state where the sheet-shaped cell culture is interposed. The small intestine may be a duodenum or a jejunum.

In the invention, the phrase "the pancreatic duct is connected to the through hole in the intestinal wall of the small intestine in a liquid-tight manner via the sheet-shaped cell culture" means that the pancreatic duct and the through hole in the intestinal wall of the small intestine are connected in close contact with each other so as to prevent the leakage of the biological fluid such as a digestive juice in the pancreas and the small intestine in the state where the sheet-shaped cell culture is interposed. That is, due to the connecting, a structure is provided in which "the pancreatic duct - the sheet-shaped cell culture - the through hole in the intestinal wall of the small intestine" are connected in close contact with one another in this order. At this time, when the pancreatic duct is exposed as a cross section of the pancreatic duct on the cut surface of the pancreas, and the cross section is constituted by a wall of the pancreatic duct and a lumen portion, all of the wall of the pancreatic duct and the lumen portion are covered with the sheet-shaped cell culture. Further, the connecting may be performed using any medical instrument or medical device such as a suture thread, a medical stapler or a suture adhesive, and from the viewpoint of liquid-tightness, the connecting is preferably performed using a suture thread. For example, the pancreatic duct and the through hole in the intestinal wall of the small intestine are sutured together with the sheet-shaped cell culture using a suture thread at the portion, the edge portion of the surface, or the like where the pancreatic duct and the through hole are connected, and are connected in close contact with each other so as to prevent the leakage of the biological fluid such as a digestive juice in the pancreas and the small intestine in the state where the sheet-shaped cell culture is interposed. At this time, the pancreatic duct and the through hole in the intestinal wall of the small intestine are connected in close contact with each other such that the cut surface of the pancreatic duct and the through hole in the intestinal wall of the small intestine are equal to each other in sizes thereof. The pancreatic duct may be a main pancreatic duct or an accessory pancreatic duct. The small intestine may be a duodenum or a jejunum.

In an embodiment, "the cut surface of the pancreas is connected to the intestinal wall of the small intestine in a liquid-tight manner via the sheet-shaped cell culture" is performed together with "the pancreatic duct is connected to a through hole in the intestinal wall of the small intestine in a liquid-tight manner via the sheet-shaped cell culture".

In an embodiment, in a state where all of the cut surface of the pancreas is covered with the sheet-shaped cell culture according to the invention, the cut surface of the pancreas and the intestinal wall of the small intestine are sutured using a suture thread at the portion, the edge portion of the surface, or the like where the cut surface and the intestinal wall of the small intestine are connected with each other, and are connected in close contact with each other so as to prevent the leakage of the biological fluid such as a digestive juice in the pancreas and the small intestine. In other words, the cut surface of the pancreas, the sheet-shaped cell culture according to the invention, and the intestinal wall of the small intestine are disposed in this order, and are fixed in a state of being connected in close contact with one another. At this time, at the portion, the edge portion of the surface, or the like where the cut surface of the pancreas and the intestinal wall of the small intestine are connected with each other, the cut surface of the pancreas and the intestinal wall of the small intestine are sutured using a suture thread together with the sheet-shaped cell culture according to the invention present between the cut surface of the pancreas and the intestinal wall of the small intestine, and are connected in close contact with each other so as to prevent the leakage of the biological fluid such as a digestive juice in the pancreas and the small intestine. The pancreatic duct may be a main pancreatic duct or an accessory pancreatic duct. The small intestine may be a duodenum or a jejunum.

In an embodiment, a position of the lumen portion of the cut surface of the pancreatic duct is disposed in accordance with the through hole in the intestinal wall of the small intestine. At this time, when the pancreatic duct is exposed as the cross section of the pancreatic duct on the cut surface of the pancreas, and the cross section is constituted by the wall of the pancreatic duct and the lumen portion, all of the wall of the pancreatic duct and the lumen portion are covered with the sheet-shaped cell culture. The pancreatic duct and the through hole in the intestinal wall of the small intestine are sutured together with the sheet-shaped cell culture using a suture thread at the portion, the edge portion of the surface, or the like where the pancreatic duct and the through hole are connected, and are connected in close contact with each other so as to prevent the leakage of the biological fluid such as a digestive juice in the pancreas and the small intestine in the state where the sheet-shaped cell culture is interposed. At this time, the pancreatic duct and the through hole in the intestinal wall of the small intestine are connected in close contact with each other such that the cut surface of the pancreatic duct and the through hole in the intestinal wall of the small intestine are equal to each other in sizes thereof. At this time, when a through hole is formed in the sheet-shaped cell culture at the lumen portion of the cut surface of the pancreatic duct due to any effect such as a physical action or a biochemical reaction, the lumina of the pancreatic duct and the small intestine communicate with each other via the through holes of the sheet-shaped cell culture and the intestinal wall of the small intestine. The pancreatic duct may be a main pancreatic duct or an accessory pancreatic duct. The small intestine may be a duodenum or a jejunum.

In the invention, the term "through hole" means a perforation penetrating from a certain site on a surface of a biological tissue to another site on a surface of the biological tissue. The through hole is, for example, a perforation penetrating from any site on an outer surface of the intestinal wall of the small intestine to any site on an inner surface of the intestinal wall of the small intestine. In an embodiment, the through hole is a perforation penetrating, in a direction perpendicular to the intestinal wall of the small intestine, the intestinal wall of the small intestine from any site on the outer surface of the intestinal wall of the small intestine. In an embodiment, the through hole is a perforation penetrating, in a direction perpendicular to the intestinal wall of the duodenum, the intestinal wall of the duodenum from any site on an outer surface of the intestinal wall of the duodenum. In an embodiment, the through hole is a perforation penetrating, in a direction perpendicular to the intestinal wall of the jejunum, the intestinal wall of the jejunum from any site on an outer surface of the intestinal wall of the jejunum. A plurality of through holes may exist in the biological tissue. The through hole may be a perforation penetrating from a site on the surface of the sheet-shaped cell culture to another site on a surface of the sheet-shaped cell culture.

The size of the through hole is not particularly limited as long as the through hole is connected to a predetermined site of the biological tissue, and for example, a diameter of the through hole is 0.5 mm to 7 mm, 0.6 mm to 6 mm, 0.7 mm to 5 mm, 0.8 mm to 4 mm, 0.9 mm to 3 mm, or 1 mm to 2 mm. The diameter of the through hole is preferably 1 mm to 2 mm from the viewpoint of the pancreatic duct being connected to the through hole in the intestinal wall of the small intestine in a liquid-tight manner.

In the invention, the phrase "a through hole is formed in the sheet-shaped cell culture in the lumen portion of the cut surface of the pancreatic duct" means that the through hole is formed in the sheet-shaped cell culture at the lumen portion of the cut surface of the pancreatic duct due to any effect such as a physical action or a biochemical reaction, so that the lumina of the pancreatic duct and the small intestine communicate with each other via the through holes of the sheet-shaped cell culture and the intestinal wall of the small intestine. The physical action means, for example, piercing, perforation, penetration of the sheet-shaped cell culture at the lumen portion of the cut surface of the pancreatic duct performed by any medical instrument or medical device such as a forceps. The biochemical reaction means, for example, lysing, degradation, and digestion of the sheet-shaped cell culture at the lumen portion of the cut surface of the pancreatic duct due to a biological fluid such as pancreatic juice. At this time, since the sheet-shaped cell culture does not come into contact with the biological fluid such as pancreatic juice at a portion except the lumen portion of the cut surface of the pancreatic duct, the lysing, degradation, digestion or the like of the sheet-shaped cell culture does not occur at a portion except the lumen portion of the cut surface of the pancreatic duct, and the sheet-shaped cell culture remains. The lysing, degradation, digestion, or the like of the sheet-shaped cell culture may be performed for several hours to several days after the transplantation of the sheet-shaped cell culture. For example, when the sheet-shaped cell culture according to the invention does not include a reinforcement layer, the lysing, degradation, digestion, or the like of the sheet-shaped cell culture may be performed for several hours after the transplantation of the sheet-shaped cell culture. For example, when the sheet-shaped cell culture according to the invention includes the reinforcement layer containing a gel and/or a polymer, the lysing, degradation, digestion, or the like of the sheet-shaped cell culture may be performed for several hours to several days after the transplantation of the sheet-shaped cell culture. The pancreatic duct may be a main pancreatic duct or an accessory pancreatic duct. The small intestine may be a duodenum or a jejunum.

In an embodiment, the pancreatic duct and the through hole in the intestinal wall of the small intestine are connected in close contact with each other so as to prevent the leakage of the biological fluid such as a digestive juice in the pancreas and the small intestine in the state where the sheet-shaped cell culture is interposed. That is, due to the connecting, the structure is provided in which "the pancreatic duct - the sheet-shaped cell culture - the through hole in the intestinal wall of the small intestine" are connected in close contact with one another in this order. At this time, when the pancreatic duct is exposed as the cross section of the pancreatic duct on the cut surface of the pancreas, and the cross section is constituted by the wall of the pancreatic duct and the lumen portion, all of the wall of the pancreatic duct and the lumen portion are covered with the sheet-shaped cell culture. At this time, the pancreatic duct and the through hole in the intestinal wall of the small intestine are connected in close contact with each other such that the cut surface of the pancreatic duct and the through hole in the intestinal wall of the small intestine are equal to each other in sizes thereof. At this time, when a through hole is formed in the sheet-shaped cell culture at the lumen portion of the cut surface of the pancreatic duct due to any effect such as a physical action or a biochemical reaction, the lumina of the pancreatic duct and the small intestine communicate with each other via the through holes of the sheet-shaped cell culture and the intestinal wall of the small intestine. The pancreatic duct may be a main pancreatic duct or an accessory pancreatic duct. The small intestine may be a duodenum or a jejunum.

On the other hand, the sheet-shaped cell culture according to the invention may have a through hole at any position before covering the cut surface of the pancreas. At this time, the sheet-shaped cell culture is disposed such that the through hole thereof is aligned with the position of the lumen portion of the cut surface of the pancreatic duct, whereby the lumina of the pancreatic duct and the small intestine can communicate with each other via the through holes of the sheet-shaped cell culture and the intestinal wall of the small intestine. At this time, the pancreatic duct and the through hole in the intestinal wall of the small intestine are connected in close contact with each other such that the cut surface of the pancreatic duct and the through hole in the intestinal wall of the small intestine are equal to each other in sizes thereof. The pancreatic duct may be a main pancreatic duct or an accessory pancreatic duct. The small intestine may be a duodenum or a jejunum.

In an embodiment, the pancreatic duct and the through hole in the intestinal wall of the small intestine are connected in close contact with each other so as to prevent the leakage of the biological fluid such as a digestive juice in the pancreas and the small intestine in the state where the sheet-shaped cell culture is interposed. That is, due to the connecting, the structure is provided in which "the pancreatic duct - the sheet-shaped cell culture - the through hole in the intestinal wall of the small intestine" are connected in close contact with one another in this order. At this time, when the pancreatic duct is exposed as the cross section of the pancreatic duct on the cut surface of the pancreas, and the cross section is constituted by the wall of the pancreatic duct and the lumen portion, all of the wall of the pancreatic duct and the lumen portion are covered with the sheet-shaped cell culture. At this time, the sheet-shaped cell culture is a sheet-shaped cell culture that has a through hole at any position before covering the cut surface of the pancreas. At this time, the sheet-shaped cell culture is disposed such that the through hole thereof is aligned with the position of the lumen portion of the cut surface of the pancreatic duct, whereby the lumina of the pancreatic duct and the small intestine can communicate with each other via the through holes of the sheet-shaped cell culture and the intestinal wall of the small intestine. At this time, the pancreatic duct and the through hole in the intestinal wall of the small intestine are connected in close contact with each other such that the cut surface of the pancreatic duct and the through hole in the intestinal wall of the small intestine are equal to each other in sizes thereof. The pancreatic duct may be a main pancreatic duct or an accessory pancreatic duct. The small intestine may be a duodenum or a jejunum.

In the invention, the phrase "the lumina of the pancreatic duct and the small intestine communicate with each other" means a state where the lumina of the pancreatic duct and the small intestine are physically connected to each other, and the biological liquid such as pancreatic juice in the lumen of the pancreatic duct can flow into the lumen of the small intestine. The pancreatic duct may be a main pancreatic duct or an accessory pancreatic duct. The small intestine may be a duodenum or a jejunum.

In the invention, the term "surgical treatment" means performing a therapeutic treatment by incising a predetermined site of a biological tissue using a surgical device, a scalpel, or the like. Examples of the surgical treatment include laparotomy, endoscopic surgery, and pancreaticoduodenectomy, but are not limited thereto.

In the invention, the term "pancreaticoduodenectomy" means a technique for performing excision of a tumor or the like generated in a bile duct, a pancreatic head portion, a duodenum, or the like. In the same surgery, excision of a stomach, a duodenum, a bile duct, a gallbladder, a pancreatic head portion, and the like is performed, and anastomosis of the stomach, the bile duct, the pancreas and the like after the excision is performed with respect to the duodenum and the jejunum after the excision. The same surgery is carried out for about 10,000 cases per year in Japan, and the surgery is a very complicated and advanced technique and is performed for a long time, and thus there are often cases where complications occur. For example, it is known that pancreatic fistula may be caused by the occurrence of a through hole in an anastomotic site after the surgery.

In the invention, the term "pancreatic fistula" means the leakage of pancreatic juice from a pancreas, may be caused by, for example, pancreatitis or a trauma, or may be caused by the occurrence of a through hole in a suture site after a surgical treatment of the pancreas. The leaked pancreatic juice may be activated by being mixed with digestive juice and bile, and may cause an inflammation due to lysing of tissues in a vicinity of a leaking site. In addition, pancreatic fistula may cause a serious prognosis by lysing blood vessels in the vicinity of the leaking site and causing hemorrhage.

In the invention, the term "fusing" means that damaged organs or tissues are connected to each other by suturing or the like, and heal. The fusing may be fusing of the biological tissue at the suture site after the surgical treatment of the pancreas. In particular, the fusing may be fusing of the pancreas and the small intestine at the suture site after the surgical treatment of the pancreas.

For example, when the cut surface of the pancreas and the intestinal wall of the small intestine are sutured together with the sheet-shaped cell culture using a suture thread at the portion, the edge portion of the surface, or the like where the cut surface and the intestinal wall are connected, and are connected in close contact with each other so as to prevent the leakage of the biological fluid such as a digestive juice in the pancreas and the small intestine in the state where the sheet-shaped cell culture is interposed, the fusing may be fusing of the cut surface of the pancreas and the intestinal wall of the small intestine at the suture site after the surgical treatment of the pancreas. For example, when the pancreatic duct and the through hole in the intestinal wall of the small intestine are sutured together with the sheet-shaped cell culture using a suture thread at the portion, the edge portion of the surface, or the like where the pancreatic duct and the through hole are connected, and are connected in close contact with each other so as to prevent the leakage of the biological fluid such as a digestive juice in the pancreas and the small intestine in the state where the sheet-shaped cell culture is interposed, the fusing may be fusing of the pancreatic duct and the through hole in the intestinal wall of the small intestine at the suture site after the surgical treatment of the pancreas. The pancreatic duct may be a main pancreatic duct or an accessory pancreatic duct. The small intestine may be a duodenum or a jejunum.

According to the invention, the cut surface of the pancreas is connected to the intestinal wall of the small intestine in a liquid-tight manner via the sheet-shaped cell culture, and fuses, or the pancreatic duct is connected to the through hole in the intestinal wall of the small intestine in a liquid-tight manner via the sheet-shaped cell culture, and fuses, whereby the liquid-tightness of the suture site of the lumina after the surgical treatment of the pancreas can be maintained. Accordingly, it is possible to prevent the occurrence of the through hole at the suture site, and thus it is possible to prevent or treat the leakage of the biological liquid such as a digestive juice flowing through the lumina from the suture site to the outside of the pancreas or the small intestine. In particular, according to the invention, it is possible to maintain the liquid-tightness of the suture site of the lumina after the treatment of pancreaticoduodenectomy. Accordingly, it is possible to prevent the occurrence of the through hole at the suture site, and thus it is possible to prevent or treat the leakage of the biological liquid such as a digestive juice flowing through the lumina from the suture site to the outside of the pancreas or the small intestine.

In addition, according to the invention, by using the sheet-shaped cell culture, it is possible to promote the fusing of the biological tissues at the suture site after the surgical treatment of the pancreas.

In the invention, the term "adhesion" means that originally separated organs or tissues are connected to each other due to a trauma, an inflammation or the like. For example, it is known that when an adhesion occurs due to laparotomy, an inflammation such as appendicitis or endometriosis, or the like, complications such as ileus, infertility, and chronic pelvic pain may occur.

In the invention, the phrase "site other than the suture site in an abdominal cavity" means any site in the abdominal cavity other than the suture site of the pancreas and the small intestine, and means any site of the originally separated organs or tissues. For example, when the cut surface of the pancreas is connected to the intestinal wall of the small intestine in a liquid-tight manner via the sheet-shaped cell culture, and fuses, the site other than the suture site in the abdominal cavity is any site in the abdominal cavity other than the suture site of the cut surface of the pancreas and the intestinal wall of the small intestine (for example, any site of a wall of the abdominal cavity, the small intestine, or the large intestine). For example, when the pancreatic duct is connected to the through hole in the intestinal wall of the small intestine in a liquid-tight manner via the sheet-shaped cell culture, and fuses, the site other than the suture site in the abdominal cavity is any site in the abdominal cavity other than the suture site of the pancreatic duct and the through hole in the intestinal wall of the small intestine (for example, any site of the wall of the abdominal cavity, the small intestine, or the large intestine).

In the invention, the phrase "adhesion at a site other than the suture site in an abdominal cavity" means the connecting at any site in the abdominal cavity other than the suture site of the pancreas and the small intestine, that is, any site of the originally separated organs or tissues. For example, when the cut surface of the pancreas is connected to the intestinal wall of the small intestine in a liquid-tight manner via the sheet-shaped cell culture, and fuses, the adhesion at the site other than the suture site in the abdominal cavity is the connecting at the suture site of the cut surface of the pancreas and the intestinal wall of the small intestine with respect to any site in the abdominal cavity other than the suture site of the cut surface of the pancreas and the intestinal wall of the small intestine (for example, any site of the wall of the abdominal cavity, the small intestine, or the large intestine). For example, when the pancreatic duct is connected to the through hole in the intestinal wall of the small intestine in a liquid-tight manner via the sheet-shaped cell culture, and fuses, the adhesion at the site other than the suture site in the abdominal cavity is the connecting at the suture site of the pancreatic duct and the through hole in the intestinal wall of the small intestine with respect to any site in the abdominal cavity other than the suture site of the pancreatic duct and the through hole in the intestinal wall of the small intestine (for example, any site of the wall of the abdominal cavity, the small intestine, or the large intestine).

According to the invention, since not only pancreatic fistula can be prevented or treated, but also no adhesion occurs at the site other than the suture site in the abdominal cavity and no inflammation occurs, a prognosis after the surgical treatment of the pancreas can be improved.

### [Method for Preventing or Treating Pancreatic Fistula]

Another aspect of the invention relates to a method for preventing or treating pancreatic fistula, and the method (sometimes referred to as "the treatment method according to the invention") includes: a step of covering, using a sheet-shaped cell culture, a cut surface of a pancreas that includes cut surfaces of a pancreatic parenchyma and a pancreatic duct; and a step of connecting the cut surface of the pancreas to an intestinal wall of a small intestine in a liquid-tight manner via the sheet-shaped cell culture.

In an embodiment, the treatment method according to the invention further includes a step of connecting the pancreatic duct to a through hole in the intestinal wall of the small intestine in a liquid-tight manner via the sheet-shaped cell culture.

In an embodiment, the treatment method according to the invention further includes a step of, by forming a through hole in the sheet-shaped cell culture at a lumen portion of the cut surface of the pancreatic duct, communicating lumina of the pancreatic duct and the small intestine with each other via the through holes in the sheet-shaped cell culture and the intestinal wall of the small intestine.

In an embodiment, the step of covering, using the sheet-shaped cell culture, the cut surface of the pancreas that includes the cut surfaces of the pancreatic parenchyma and the pancreatic duct is performed by a transplantation device.

In the invention, the term "transplantation device" means any medical device that can cover, using the sheet-shaped cell culture, the cut surface of the pancreas that includes the cut surfaces of the pancreatic parenchyma and the pancreatic duct. The transplantation device is, for example, a medical device having a planar structure that is capable of supporting a sheet-shaped cell culture and detaching the sheet-shaped cell culture, and is preferably in a form that can be inserted into a tubular body transabdominally inserted into a body-cavity in endoscopic surgery from the viewpoint of operating in the abdominal cavity. [Method for Producing Sheet-shaped Cell Culture according to Invention]

Another aspect of the invention relates to a method for producing the sheet-shaped cell culture according to the invention, and the method (sometimes referred to as "the production method according to the invention") includes: (i) a step of seeding a cell population containing sheet-forming cells on a culture substrate; (ii) a step of forming the cell population seeded in step (i) into a sheet, and forming a sheet-shaped cell culture; and (iii) a step of detaching the sheet-shaped cell culture formed in step (ii) from the culture substrate. [Composition Containing Sheet-shaped Cell Culture according to Invention and The Like]

Another aspect of the invention relates to a composition (for example, a pharmaceutical composition), a graft, a medical product, and the like (sometimes referred to as "the composition and the like according to the invention"), which contain the sheet-shaped cell culture according to the invention.

The composition and the like according to the invention can contain various additional components in addition to the sheet-shaped cell culture according to the invention. The additional components are, for example, a pharmaceutically acceptable carrier, components that enhance viability, engraftment and/or function of the sheet-shaped cell culture, active components useful for regeneration, healing and/or fusing of a biological tissue and useful in promoting the same, and/or grafts. A person skilled in the art is familiar with these additional components, and can appropriately select and use any known additional component. In addition, the composition and the like according to the invention can be used together with the additional components in addition to the sheet-shaped cell culture according to the invention.

In an embodiment, the composition and the like according to the invention is used for covering the cut surface of the pancreas.

In an embodiment, the composition and the like according to the invention is used for preventing or treating pancreatic fistula. [Use in Manufacture of Medicament for Preventing or Treating Pancreatic Fistula]

Another aspect of the invention relates to use of the sheet-shaped cell culture according to the invention or the composition and the like according to the invention in manufacture of a medicament for preventing or treating pancreatic fistula (sometimes referred to as "the use in the manufacture of the medicament according to the invention").

### [Use for Preventing or Treating Pancreatic Fistula]

Another aspect of the invention relates to use of the sheet-shaped cell culture according to the invention or the composition and the like according to the invention for preventing or treating pancreatic fistula (sometimes referred to as "the use according to the invention").

The invention is explained in more detail with reference to the following examples, but the examples are specific examples of the invention and the invention is not limited to the examples.

### [Examples]

### Example 1: Preparation of Sheet-shaped Cell Culture

### (1) Preparation of Cells

The striated muscle of the porcine lower limb was collected under general anesthesia, and the collected tissue was treated with an enzyme digestive solution containing collagenase and trypsin, and was dispersed in single cells. The cells were cultured under the conditions of 37°C and 5% CO₂ in a MCDB131 medium containing 20% FBS until the cells reached confluent.

### (2) Preparation of Sheet-shaped Cell Culture of Pig Skeletal myoblast cells

The cells cultured in (1) were recovered on the day of pancreaticoduodenectomy to be described in Example 2, and the recovered cells were seeded in a temperature-responsive culture dish of 60 mm (UpCell (registered trademark), manufactured by Cell Seed) in an amount of 2.2×10⁷ cells, and were cultured in a DMEM/F12 medium containing 20% FBS for 12 hours. Thereafter, the temperature was lowered to 20°C, and an autologous sheet-shaped cell culture (a sheet-shaped cell culture of pig skeletal myoblast cells) was detached from the temperature-responsive culture dish and recovered (FIG. 1). A fibrin gel was added in to the recovered sheet-shaped cell culture to form a reinforcement layer containing the fibrin gel.

### Example 2: Prevention of Pancreatic Fistula by Sheet-shaped Cell Culture

### (1) Pancreaticoduodenectomy of Control Group

Pancreaticoduodenectomy was performed on the pig, from which the striated muscle was collected in Example 1, under general anesthesia to obtain a control group (n = 9). The cut surface of the pancreas of the control group included the cut surfaces of the pancreatic parenchyma and the pancreatic duct. The cut surface of the pancreas of the control group was directly sutured to the intestinal wall of the duodenum without using the sheet-shaped cell culture.

Specifically, the pancreatic head portion and the anus-side piece of the duodenal papilla portion were subjected to complication ablation, duodenum stumps were anastomosed in an end-to-end manner, and the distal duodenum and the pancreatic parenchyma were anastomosed (FIG. 2). More specifically, 2 cm of an oral-side piece and 2 cm of the anus-side piece from the duodenal papilla portion and the pancreatic head portion were subjected to complication ablation, and cut duodenum stumps were anastomosed using a 4-0 synthetic absorbable monofilament suture thread with a needle in an end-to-end manner by Gambee method. Then, the pancreas and the duodenum were subjected to pressure-bonding anastomosis (excluding pancreatic duct anastomosis) using a 4-0 synthetic absorbable monofilament suture thread with needles at both ends by Blumgart-dumpling method. A CV catheter and an ascites drain were indwelled in the pig for a clinical chemistry test.

### (2) Pancreaticoduodenectomy of Sheet Treatment Group

Pancreaticoduodenectomy was performed on the pig, from which the striated muscle was collected in Example 1, under general anesthesia by using the sheet-shaped cell culture prepared in Example 1 to obtain a sheet treatment group (n = 5). The cut surface of the pancreas of the sheet treatment group included the cut surfaces of the pancreatic parenchyma and the pancreatic duct. The sheet-shaped cell culture prepared in Example 1 was transplanted to cover all of the cut surface of the pancreas of the sheet treatment group. After several minutes from the transplantation, the cut surface of the pancreas was sutured to the intestinal wall of the duodenum via the sheet-shaped cell culture.

Specifically, the pancreatic head portion and the anus-side piece of the duodenal papilla portion were subjected to complication ablation, the duodenum stumps were anastomosed in an end-to-end manner, and after the sheet-shaped cell culture was transplanted to a pancreatic head incision surface (the region surrounded by the dotted line in A in FIG. 3), the distal duodenum and the pancreatic parenchyma were anastomosed (B in FIG. 3). More specifically, 2 cm of the oral-side piece and 2 cm of the anus-side piece from the duodenal papilla portion and the pancreatic head portion were subjected to complication ablation, and the cut duodenum stumps were anastomosed using a 4-0 synthetic absorbable monofilament suture thread with a needle in an end-to-end manner by Gambee method. Then, the sheet-shaped cell culture was transplanted to a pancreas incision surface (the pancreatic duct was exposed by cutting). Further, the pancreas and the duodenum were subjected to pressure-bonding anastomosis (excluding pancreatic duct anastomosis) using a 4-0 synthetic absorbable monofilament suture thread with needles at both ends by Blumgart-dumpling method. A CV catheter and an ascites drain were indwelled in the pig for a clinical chemistry test.

### (3) Histopathologic Test after Pancreaticoduodenectomy

After 3 days from the transplantation of the sheet-shaped cell culture, the control group and the sheet treatment group were subjected to laparotomy again under general anesthesia, the anastomotic site of the duodenum and the pancreatic parenchyma was resected in the control group, the transplanted site of the sheet-shaped cell culture (the suture site between the cut surface of the pancreas and the intestinal wall of the duodenum) was resected in the sheet treatment group, and the anastomotic site and the transplanted site were subjected to a histopathologic test. A in FIG. 4 shows an HE staining image (x200) of the anastomotic site of the duodenum and the pancreatic parenchyma resected 3 days after pancreaticoduodenectomy, of the control group. Arrows indicate hemorrhage and concentrated proteins. B and C in FIG. 4 show HE staining images (x200) of the transplanted site of the sheet-shaped cell culture resected 3 days after the transplantation of the sheet-shaped cell culture, of the sheet treatment group. A region sandwiched by dotted lines indicates the sheet-shaped cell culture. D in FIG. 4 shows an anti-DESMIN antibody immunostaining image (x200) of the transplanted site of the sheet-shaped cell culture resected 3 days after the transplantation of the sheet-shaped cell culture, of the sheet treatment group. A region sandwiched by dotted lines indicates the sheet-shaped cell culture.

As a result, it was found that in the transplanted site of the resected sheet-shaped cell culture, the sheet-shaped cell culture remains in a state of being in close contact with the pancreatic parenchyma (a part of the cut surface of the pancreas) and the duodenum (a part of the intestinal wall of the duodenum) (the regions sandwiched by the dotted lines in B to D in FIG. 4). In addition, adhesion at sites other than the transplanted site of the sheet-shaped cell culture did not occur, and the inflammation did not occur (not shown). In B to D in FIG. 4, a uniform layer adjacent to the sheet-shaped cell culture is a reinforcement layer containing the fibrin gel.

### (4) Clinical Chemistry Test after Pancreaticoduodenectomy

In the control group and the sheet treatment group, venous blood was collected via the CV catheter indwelled in the pig 0, 1, and 3 days after pancreaticoduodenectomy, and serum was prepared by a common method. Further, in the control group and the sheet treatment group, ascites was collected via the ascites drain indwelled in the pig 1 and 3 days after pancreaticoduodenectomy. The serum and the ascites were supplied for the clinical chemistry test, and amylase values and lipase values thereof were measured.

As a result, there was a tendency that as compared with the control group (n = 9), the amylase value of the ascites and the lipase value of the ascites decreased in the sheet treatment group (n = 5) 3 days after pancreaticoduodenectomy (not shown). Further, FIG. 5 shows (the amylase value of the ascites)/ (the amylase value of the serum) (A in FIG. 5) and (the lipase value of the ascites)/ (the lipase value of the serum) (B in FIG. 5) obtained 3 days after pancreaticoduodenectomy in accordance with a clinical evaluation method for human pancreatic fistula. In addition, there was a tendency that as compared with the control group (n = 9), the amylase value of the ascites and the lipase value of the ascites decreased in the sheet treatment group (n = 5) even 1 day after pancreaticoduodenectomy (not shown).

From the above results, according to the invention, it is possible to maintain the liquid-tightness of the suture site after the surgical treatment of the pancreas, and it is possible to prevent or treat pancreatic fistula. In particular, according to the invention, it is possible to maintain the liquid-tightness of the suture site after the treatment of pancreaticoduodenectomy, and it is possible to prevent or treat pancreatic fistula. Further, according to the invention, since not only pancreatic fistula can be prevented or treated, but also no adhesion occurs at a site other than the suture site in the abdominal cavity and no inflammation occurs, the prognosis after the surgical treatment of the pancreas can be improved. In addition, according to the invention, by using the sheet-shaped cell culture, it is also possible to promote the fusing of the biological tissues at the suture site after the surgical treatment of the pancreas.

## Claims

1. A sheet-shaped cell culture for covering a cut surface of a pancreas, wherein
the cut surface of the pancreas includes cut surfaces of a pancreatic parenchyma and a pancreatic duct, and is connected to an intestinal wall of a small intestine in a liquid-tight manner via the sheet-shaped cell culture.

2. The sheet-shaped cell culture according to claim 1, wherein
the pancreatic duct is connected to a through hole in the intestinal wall of the small intestine in a liquid-tight manner via the sheet-shaped cell culture.

3. The sheet-shaped cell culture according to claim 2, wherein
by forming a through hole in the sheet-shaped cell culture at a lumen portion of the cut surface of the pancreatic duct, lumina of the pancreatic duct and the small intestine communicate with each other via the through holes in the sheet-shaped cell culture and the intestinal wall of the small intestine.

4. The sheet-shaped cell culture according to any one of claims 1 to 3, wherein
the pancreatic duct is a main pancreatic duct and/or an accessory pancreatic duct.

5. The sheet-shaped cell culture according to any one of claims 1 to 4, wherein
the small intestine is a duodenum or a jejunum.

6. The sheet-shaped cell culture according to any one of claims 1 to 5, wherein
the cut surface of the pancreas, the through hole in the intestinal wall of the small intestine, and/or the through hole in the sheet-shaped cell culture is formed by a surgical treatment.

7. The sheet-shaped cell culture according to claim 6, wherein
the surgical treatment is pancreaticoduodenectomy.

8. The sheet-shaped cell culture according to any one of claims 1 to 7, wherein
the sheet-shaped cell culture is used for preventing or treating pancreatic fistula.

9. The sheet-shaped cell culture according to any one of claims 1 to 8, further comprising:
skeletal myoblast cells.

10. The sheet-shaped cell culture according to any one of claims 1 to 9, further comprising:
a reinforcement layer containing a gel and/or a polymer.

11. A method for preventing or treating pancreatic fistula, the method comprising:
a step of covering, using a sheet-shaped cell culture, a cut surface of a pancreas that includes cut surfaces of a pancreatic parenchyma and a pancreatic duct; and
a step of connecting the cut surface of the pancreas to an intestinal wall of a small intestine in a liquid-tight manner via the sheet-shaped cell culture.

12. The method according to claim 11, further comprising:
a step of connecting the pancreatic duct to a through hole in the intestinal wall of the small intestine in a liquid-tight manner via the sheet-shaped cell culture.

13. The method according to claim 12, further comprising:
a step of, by forming a through hole in the sheet-shaped cell culture at a lumen portion of the cut surface of the pancreatic duct, communicating lumina of the pancreatic duct and the small intestine with each other via the through holes in the sheet-shaped cell culture and the intestinal wall of the small intestine.

14. The method according to any one of claims 11 to 13, wherein
the step of covering, using the sheet-shaped cell culture, the cut surface of the pancreas that includes the cut surfaces of the pancreatic parenchyma and the pancreatic duct is performed by a transplantation device.
